# EUROPÄISCHE PATENTSCHRIFT

(11) **EP 1 993 983 B1**
(45) Veröffentlichungstag und Bekanntmachung des Hinweises auf die Patenterteilung: **23.09.2015**
(21) Anmeldenummer: 07712307.3
(22) Anmeldetag: 23.02.2007
(51) Int. Cl.: C07C 29/141, C07C 31/20

(54) **VERFAHREN ZUR HYDRIERUNG VON METHYLOLALKANALEN**
PROCESS FOR HYDROGENATING METHYLOLALKANALS
PROCEDE D'HYDROGENATION DE METHYLOL ALCANALS

(30) Priorität: 01.03.2006 DE 102006009838
(43) Veröffentlichungstag der Anmeldung: 26.11.2008
(73) Patentinhaber: BASF SE, 67056 Ludwigshafen (DE)
(72) Erfinder: MAAS, Steffen, 55270 Bubenheim (DE); JOHANN, Thorsten, 67063 Ludwigshafen (DE); KOCH, Michael, 68163 Mannheim (DE); SIRCH, Tilman, 67105 Schifferstadt (DE); SCHLITTER, Stephan, 67117 Limburgerhof (DE); RITTINGER, Stefan, 68199 Mannheim (DE); STEINIGER, Michael, 67435 Neustadt (DE)
(86) Internationale Anmeldenummer: PCT/EP2007/051760
(87) Internationale Veröffentlichungsnummer: WO 2007/099064

(56) Entgegenhaltungen:
- EP-B1- 0 759 896
- WO-A-2004/092097

## Beschreibung

Die Erfindung betrifft ein Verfahren zur katalytischen Hydrierung von Methylolalkanalen in der Flüssigphase an einem Hydrierkatalysator durch pH-Wert-Kontrolle des Hydrieraustrags.

Die katalytische Hydrierung von Carbonylverbindungen wie z.B. Aldehyden zur Herstellung einfacher und funktionalisierter Alkohole nimmt in den Produktionssträngen der chemischen Grundstoffindustrie eine bedeutende Stellung ein. Besonders gilt dies für die Hydrierung von Aldehyden, die über Oxosynthese oder Aldolreaktion zugänglich sind.

Durch Aldolreaktion von Alkanalen mit überschüssigem Formaldehyd in Gegenwart stöchiometrischer Mengen an Base werden Methylolalkanale zugänglich. Aus WO 01/51438 ist es bekannt, anorganische Hydroxide wie Nariumhydroxid oder Calciumhydroxid als Base einzusetzen. WO 98/28253 und DE-A 1957591 beschreiben Amine als basische Katalysatoren der Aldolisierung und WO 98/29374 basische Ionentauscher. Das Methylolalkanal wird nach diesen Verfahren als 20 bis 80 Gew.-%ige wässrige Lösung erhalten. Der pH-Wert dieser wässrigen Lösung liegt nur bei 3,5 bis 6,0, da der basische Katalysator der Aldolisierung auch die Cannizzaro-Reaktion des Formaldehyds zu Ameisensäure katalysiert, die wiederum die Base zumindestens teilweise neutralisiert.

Will man mehrwertige Alkohole wie Pentaerythrit, Neopentylglykol oder Trimethylolpropan aus wässrigen Methylolalkanal-Lösungen herstellen, müssen diese Lösungen hydriert werden.

Diese Hydrierung wird im allgemeinen bei Temperaturen von über 80°C durchgeführt. Es werden Rückspaltungen der Methylolgruppe zu freiem Aldehyd, die Cannizzaro-Reaktion des Formaldehyds zu Ameisensäure und darüber hinaus Ether-, Ester- und Acetalbildung im Hydrierreaktor beobachtet. Diese Nebenreaktionen führen zu niedrigen Hydrierselektivitäten und zu niedrigen Ausbeuten an mehrwertigem Alkohol.

Zudem sind viele Hydrierkatalysatoren unter diesen Bedingungen nicht stabil. Insbesondere Katalysatoren auf Basis der Oxide des Aluminiums und Siliziums wie sie aus EP-A 44 444 und WO 95/32171 bekannt sind, verlieren in Gegenwart dieser wässrigen Methylolalkanallösungen unter Hydrierbedingungen an Aktivität, was erfahrungsgemäß über die Dauer von einigen Monaten zu einem deutlich geringeren Umsatz führt. Großtechnisch könnte dies zumindest teilweise dadurch kompensiert werden, dass die Hydriertemperatur schrittweise erhöht wird. Neben dem unwirtschaftlichen erhöhten Energieverbrauch den diese Maßnahme bedeutet, nehmen andererseits ab einer bestimmten Temperatur Nebenreaktionen stark zu, die zu erhöhten Einsatzzahlen (Verbrauch der Einsatzstoffe) oder zu unreinerem Produkt führen, so dass der Katalysator durch einen neuen ersetzt werden muss.

So findet beispielsweise bei der Hydrierung von Hydroxypivalinaldehyd oder von Dimethylolbutanal zu den entsprechenden Alkoholen Neopentylglykol (NPG) und Trimethylolpropan (TMP) mit zunehmender Temperatur eine Retroaldolreaktion statt. Die dabei entstehenden Aldehyde werden zu unerwünschten Nebenprodukten hydriert (bei der NPG-Herstellung entstehen so Isobutanol und Methanol, bei der TMP-Herstellung 2-Methylbutanol, 2-Ethyl-1,3-propandiol und Methanol) und die Ausbeute wird entsprechend vermindert. Im Falle der NPG-Synthese wird bei erhöhter Temperatur auch die Bildung des cyclischen Acetals von NPG und Hydroxypivalinaldehyd (HPA) verstärkt beobachtet. Dieses Nebenprodukt ist destillativ nicht von NPG zu trennen und führt daher zu einem unreineren Wertprodukt. Weiterhin fördern hohe Temperaturen die thermische Tischtschenko-Reaktion von HPA zu Hydroxypivalinsäureneopentylglykolester (HPN). Dieser wird teilweise zu NPG und Hydroxypivalinsäure (HPS) hydrolysiert, was wiederum zur Absenkung des pH-Wertes führt. Aufgrund dieser Nebenreaktionen ist die Temperaturerhöhung als Mittel zum Konstanthalten der Hydrieraktivität eines alternden Katalysators durch wirtschaftliche Faktoren wie Ausbeute und Produktreinheit limitiert.

Erfindungsgemäß wurde nun festgestellt, dass der pH-Wert im Hydrierreaktor entscheidenden Einfluß auf das Hydrierergebnis und die Katalysatoraktivität hat. Der pH-Wert im Hydrierreaktor wird entscheidend durch dessen Ameisensäuregehalt bestimmt. Einen Einfluss auf den pH-Wert hat weiterhin auch die Hydrolyse von HPN zu NPG und HPS.

Ameisensäure, die bei der Aldolisierung als Nebenprodukt über eine Cannizzaro-Reaktion aus Formaldehyd gebildet wurde, wird im Laufe der großtechnisch betriebenen Hydrierung zu CO₂ und H₂ bzw. zu CO und H₂O zersetzt. CO und CO₂ lassen sich im Abgas der Hydrierung nachweisen. Die Zersetzungsrate des unerwünschten Nebenprodukts Ameisensäure hängt neben der Temperatur entscheidend vom Alter des Katalysators ab. Mit zunehmendem Alter des Katalysators nimmt auch die Zersetzungsrate von Ameisensäure unter konstanten Reaktionsbedingungen permanent ab.

Im Allgemeinen wird der Hydrierreaktor großtechnisch, um eine gute Abfuhr der Hydrierwärme zu gewährleisten, mit einem hohen Kreislaufverhältnis betrieben, das heißt die Kreislaufmenge wird größer gewählt als die Menge an Frischzulauf (Zulauf des Frischfeeds). Daher entspricht der pH-Wert im Reaktor dem pH-Wert des Hydrieraustrags, liegt aber deutlich höher als der pH-Wert des Hydrierzulaufs. Die Differenz der pH-Werte zwischen Zulauf (Feed) und Austrag wird durch die Aktivität des Katalysators bezüglich der Zersetzung von Ameisensäure, sowie durch Temperatur, Abgasmenge und Belastung bestimmt.

Aus dem Stand der Technik sind bereits Verfahren bekannt, bei denen versucht wurde über den pH-Wert das Hydrierergebnis zu beeinflussen.

PCT/WO 2004/092097 beschreibt ein Hydrierverfahren bei dem der Hydrierfeed durch Zusatz einer Base neutralisiert wird mit dem Ziel, negative Auswirkungen des pH-Wertes auf die mechanische Stabilität des Katalysatorträgers zu verhindern. Nachteilig an diesem Verfahren ist es, dass durch die Einstellung eines pH-Wertes im Hydrierzulauf aufgrund der oben beschriebenen Effekte wie der Zersetzung von Ameisensäure oder der Bildung von Hydroxypivalinsäure eine effektive pH-Wert-Kontrolle im Hydrierreaktor nicht möglich ist. Die sich bei dieser Fahrweise zwangsläufig ergebenden Schwankungen des pH-Werts im Hydrierreaktor verhindern einen optimalen Hydrierumsatz und das Erreichen einer optimale Selektivität.

Die JP 2004-182622 beschreibt ein Hydrierverfahren bei dem der pH-Wert auf pH 5,5 - 7,5 im Hydrierfeed eingestellt wird. Bei kleineren pH-Werten wurde ein Austragen des Aktivmetalls aus dem Katalysator beobachtet, was zu einem kontinuierlichen Aktivitätsverlust führte. Zudem können die Metallspuren in der weiteren Aufarbeitung stören. Bei höheren pH-Werten wurden Aldolkondensationen beobachtet, die die Selektivität des Verfahrens schmälern.

Der Erfindung lag daher die Aufgabe zugrunde, ein Verfahren zur katalytischen Hydrierung von Methylolalkanalen bereitzustellen, bei dem mehrwertige Alkohole mit guten Hydrierselektivitäten und Ausbeuten bei hohen Standzeiten des Katalysators zugänglich gemacht werden können.

Diese Aufgabe wird gelöst durch ein Verfahren zur katalytischen Hydrierung von Methylolalkanalen der allgemeinen Formel 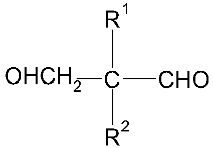 in der R¹ und R² unabhängig voneinander eine weitere Methylolgruppe oder eine Alkylgruppe mit 1 bis 22 C-Atomen oder eine Aryl- oder Aralkylgruppe mit 6 bis 33 C-Atomen bedeuten, in der Flüssigphase an einem Hydrierkatalysator, dadurch gekennzeichnet, dass durch Zusatz mindestens eines tertiären Amins, einer anorganischen Base oder einer anorganischen oder organischen Säure zum Hydrierfeed ein pH-Wert von 7,0 bis 9,0 im Hydrieraustrag eingestellt wird.

Das erfindungsgemäße Verfahren ermöglicht eine effektive pH-Wert-Kontrolle im Reaktor durch das Einstellen des erfindungsgemäßen pH-Wertbereichs im Hydrieraustrag. pH-Wert-Schwankungen durch Nebenreaktionen der Hydrierung und der Einfluss eines alternden Katalysators können vermieden werden. Hohe Umsätze, Selektivitäten und Standzeiten des Katalysators werden erreicht.

Unter Hydrierfeed wird in dieser Anmeldung eine Methylolalkanal der allgemeinen Formel I enthaltende wässrige Lösung, insbesondere eine 20 bis 80 Gew.-% Methylolalkanal enthaltende wässrige Lösung, verstanden. Ein solcher Hydrierfeed wird bevorzugt gemäß WO 98/28253 oder DE-A 1957591 durch Umsetzung von Aldehyden mit Formaldehyd hergestellt.

Es wird dabei so verfahren, dass der Aldehyd mit der 1- bis 8-fachen Mengen Formaldehyd in Gegenwart eines tertiären Amins (Aldolisierung) umgesetzt wird, und man das so erhaltene Reaktionsgemisch in zwei Lösungen auftrennt, wobei eine Lösung das erwähnte Methylolalkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Methylolalkanal enthaltende wässrige Lösung kann als Hydrierfeed in dem erfindungsgemäßen Verfahren eingesetzt werden.

Es ist jedoch auch möglich die wässrige Methylolalkanallösung als Hydrierfeed nach anderen Verfahren des Standes der Technik, beispielsweise nach den aus WO 01/51438, WO 97/17313 und WO 98/29374 bekannten Verfahren herzustellen.

In einer bevorzugten Variante des erfindungsgemäßen Verfahrens wird eine besonders Formaldehyd-arme oder Formaldehyd-freie wässrige Methylolalkanallösung als Hydrierfeed eingesetzt. In einer Formaldehyd-armen Methylolalkanallösung liegt der Gehalt an Formaldehyd unter 5 Gew.-%. Die Abtrennung von Formaldehyd aus dem Aldolisierungsaustrag, der beispielsweise gemäß WO 98/28253 erhalten wurde, kann nach den aus dem Stand der Technik bekannten Verfahren, beispielsweise durch Destillation, erfolgen.

Bei dem Methylolalkanal der allgemeinen Formel I handelt es sich bevorzugt um Dimethylolalkanal, Pentaerythrose oder Hydroxypivalinaldehyd.

Der Hydrierfeed wird vor dem Hydrierreaktoreingang mit tertiärem Amin, anorganischer Base oder anorganischer oder organischer Säure vermischt bis der Hydrieraustrag, der nach dem Reaktorausgang entnommen wird, einen pH-Wert von 7,0 bis 9,0, für die Herstellung von Neopentylglykol bevorzugt einen pH-Wert von 8,0 bis 9,0 , für die Herstellung von Trimethylolpropan bevorzugt einen pH-Wert von 7,0 bis 8,0, aufweist. Es ist auch möglich, den Hydrierfeed und das tertiäre Amin, die anorganische Base oder die anorganische oder organische Säure getrennt in den Reaktor einzuspeisen und dort zu vermischen.

Als geeignete tertiäre Amine werden die in DE-A 25 07 461 aufgeführten Amine beispielhaft genannt. Als tertiäre Amine sind Tri-n-C₁-bis C₄-alkylamine bevorzugt und Trimethylamin, Triethylamin, Tri-n-propylamin und Tri-n-butylamin besonders bevorzugt. Im Allgemeinen werden im erfindungsgemäßen Verfahren bis zu 10 Gew.-% (bezogen auf den Hydrierfeed) des tertiären Amins zur pH-Kontrolle zugesetzt. Das Amin kann als Reinsubstanz oder als wässrige Lösung eingesetzt werden.

Geeignete anorganische Basen sind die Carbonate, Hydrogencarbonate und Hydroxide der Alkali- und Erdalkalimetalle.

Amine sind besonders vorteilhaft zur pH-Einstellung zu verwenden, da sie mit Ameisensäure thermisch zersetzbare Salze bilden, welche nach der Hydrierung wieder gespalten werden können. Somit kann ein Salzanfall vermieden werden und das tertiäre Amin kann in den Prozess rückgeführt werden.

Besonders vorteilhaft ist die Verwendung desselben tertiären Amins im Aldolisierungsprozess zum Methylolalkanal - der Kondensation von höherem Aldehyd und Formaldehyd - und in der Hydrierung.

Als anorganische oder organische Säuren können erfindungsgemäß Mineralsäuren wie Salzsäure, Schwefelsäure oder Phosphorsäure oder organische Säuren wie Zitronensäure, Essigsäure oder Ethylhexansäure verwendet werden. Bevorzugt wird Essigsäure verwendet. Im Allgemeinen werden 0 und 3 Gew.% (bezogen auf den Hydrierfeed) einer 10%igen wässrigen Lösung der Säure zur pH-Kontrolle zugesetzt.

Die Messung des pH-Werts erfolgt mit den bekannten Techniken, vorzugsweise mit einer Glaselektrode und einem pH-Meter.

Erfindungsgemäß verwendbare Katalysatoren sind für Hydrierungen geeignete Katalysatoren, die bevorzugt mindestens ein Metall der Nebengruppe 8 bis 12 des Periodensystems der Elemente wie Fe, Ru, Os, Co, Rh, Ir, Ni, Pd, Pt, Cu, Ag, Au, Zn, Cd, Hg, bevorzugt Fe, Co, Ni, Cu, Ru, Pd, Pt, besonders bevorzugt Cu, bevorzugt auf einem üblichen Trägermaterial, besonders bevorzugt auf einem aus den Oxiden des Titans, Zirkoniums, Hafniums, Siliziums und/oder Aluminiums aufweisen. Die Herstellung der erfindungsgemäß verwendbaren Katalysatoren kann nach aus dem Stand der Technik bekannten Verfahren zur Herstellung derartigen Trägerkatalysatoren erfolgen. Bevorzugt verwendet werden können auch Trägerkatalysatoren, die Kupfer auf einem Aluminiumoxid- oder Titandioxid-haltigen Trägermaterial in An- oder Abwesenheit eines oder mehrer der Elemente Magnesium, Barium, Zink oder Chrom aufweist. Derartige Katalysatoren und ihre Herstellung sind aus WO 99/44974 bekannt.

Weiterhin sind kupferhaltige Trägerkatalysatoren wie sie z.B. in WO 95/32171 beschrieben sind und die in EP-A 44 444 und DE 19 57 591 offenbarten Katalysatoren für die erfindungsgemäße Hydrierung geeignet.

Die Hydrierung kann diskontinuierlich oder kontinuierlich durchgeführt werden z.B. in einem mit einer Katalysatorschüttung gefüllten Reaktorrohr, bei dem die Reaktionslösung über die Katalysatorschüttung z.B. in Riesel- oder Sumpffahrweise, wie in DE-A 19 41 633 oder DE-A 20 40 501 beschrieben, geleitet wird. Es kann vorteilhaft sein, einen Teilstrom des Reaktionsaustrages, gegebenenfalls unter Kühlung, zurückzuführen und wieder über das Katalysatorfestbett zu leiten. Diese Kreislauffahrweise wird bevorzugt mit einem Verhältnis von Kreislauf zu Zulauf (Feed) von 10:1 bis 20:1 betrieben. Ebenso kann es vorteilhaft sein, die Hydrierung in mehreren hintereinander geschalteten Reaktoren durchzuführen, beispielsweise in 2 bis 4 Reaktoren, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. Dabei kann es zweckmäßig sein, den Hydrieraustrag aus dem vorangehenden Reaktor vor dessen Eintritt in den nachfolgenden Reaktor zu kühlen, beispielsweise mittels Kühlvorrichtungen oder durch Eindüsen kalter Gase, wie Wasserstoff oder Stickstoff oder Einleiten eines Teilstroms an kalter Reaktionslösung.

Die Hydriertemperatur liegt im allgemeinen zwischen 50 und 180°C, bevorzugt 90 und 140°C. Als Hydrierdruck werden im allgemeinen 10 bis 250 bar, bevorzugt 20 bis 120 bar angewandt.

Die Hydrierung kann unter Zugabe eines inerten Lösungsmittels durchgeführt werden. Als Lösungsmittel sind Wasser, cyclische Ether, wie THF oder Dioxan, als auch acyclische Ether einsetzbar, ebenso niedere Alkohole z.B. Methanol, Ethanol oder 2-Ethylhexanol.

Im übrigen können beliebige Hydriermethoden angewendet und Hydrierkatalysatoren eingesetzt werden, wie sie für die Hydrierung von Aldehyden üblich und in der Standardliteratur eingehend beschrieben sind.

### Beispiele

### Beispiel 1

Hydrierung von Hydroxypivalinaldehyd zu Neopentylglykol

### Hydrierfeed

1,1 mol Isobutyraldehyd wurden mit 1 mol Formaldehyd in Form einer 40 %igen Lösung und 4 mol.-% Trimethylamin, bezogen auf Isobutyraldehyd, bei 75°C 1 h lang gerührt. Die Reaktionslösung wurde eingeengt, indem bei Normaldruck Leichtsieder wie beispielsweise Isobutyralydehyd und ein Teil des Wassers abdestilliert wurden. Der erhaltene Sumpf bestand aus 75 Gew.-% Hydroxypivalinaldehyd, 20 Gew.-% Wasser und ca. 5 Gew.-% sonstigen organischen Nebenkomponenten.

### Katalysatorherstellung

Alle unter diesem Unterpunkt angegebenen Prozentangaben stellen, soweit nicht anders vermerkt, Gew.-%e dar. Die angegebenen prozentualen Zusammensetzungen beziehen sich auf die oxidischen Bestandteile der fertigen Katalysatoren.

Einsatzstoffe waren eine 20 gew.-%ige Sodalösung und eine wässrige Lösung I die 2,67 Gew.-% Al und 5 Gew.-% Cu in Form ihrer Nitrate enthielt.
Bei der Fällung wurde in einem Fällkessel bei 80°C Lösung I und Sodalösung so eindosiert, dass sich ein pH-Wert von 5,6 einstellte.
Das Fällungsgemisch wurde in einen größeren Rührbehälter überführt und dort bei 80°C mit Sodalösung auf einen pH von 7,9 eingestellt. Sodann wurde die Suspension auf eine Filterpresse geleitet.
Das Gemisch wurde darauf hin filtriert und mit Wasser nitratfrei gewaschen. Die Filterpaste wurde in Wasser suspendiert und in einem Sprühturm mit Heißluft bei 130 - 150°C Ausgangstemperatur getrocknet. Im Anschluss erfolgt bei einer Temperatur von 375 - 390°C eine Kalzination. Anschließend wurde das Pulver mit 3 Gew.-% Graphit als Hilfsmittel zu Tabletten mit 3 x 3 mm tablettiert.
Die erhaltenen Tabletten wurden nun in einem beheizten Drehrohr bei einer Temperatur von 600°C während 60 min. calciniert.

Der Katalysator bestand aus 55 % CuO und 45 Gew.-% Al₂O₃, wies eine spezifische Oberfläche (BET) von 95 m²/g, eine Hg-Porosität von 0,38 ml/g bei einem Rüttelgewicht von 1042 g/l auf.

150 ml dieses Cu/Al₂O₃-Katalysators wurden in einem Rohrreaktor bei 190°C durch Überleiten eines Gemisches aus 5 Vol.% Wasserstoff und 95 Vol.% Stickstoff (Gesamtvolumen 50 Nl/h) drucklos 24 h aktiviert.

### Hydrierung

Als Ausgangslösung diente das vorstehend als Hydrierfeed beschriebene Gemisch. Diesem Gemisch wurden von 0 bis 7 Gew.-% (bezogen auf den Hydrierfeed) einer 15 gew.-%igen wässrigen Lösung von Trimethylamin (2 bis 5 Gew.-% (bezogen auf den Hydrierfeed) bzw. einer 5 gew.-%igen wässrigen Lösung von Zitronensäure bei den Vergleichsbeispielen) zugesetzt, um den jeweils auf den in Tabelle 1 angegebenen pH-Wert des Hydrieraustrags einzustellen. Der so erhaltene Hydrierzulauf wurde in einem Hydrierreaktor mit Flüssigkeitskreislauf (Kreislauf: Zulauf = 10:1) mit einer Katalysatorbelastung von 0,4 kg_{HPA}/lₖₐₜ x h in Rieselfahrweise bei 40 bar und 120°C über den Katalysator gepumpt.

Einen Vergleich des erfindungsgemäßen Verfahren mit den Vergleichsbeispielen V1 und V2, bei denen der pH-Wert des Hydrieraustrags jeweils außerhalb des erfindungsgemäßen Bereichs liegt, zeigt Tabelle 1.

Zur pH-Wert-Messung wurde ein pH-Meter der Firma Knick Modell 766 mit einer Glaselektrode N1041A der Firma Schott verwendet.

**Tabelle 1:**

| Beispiel | pH-Wert des Hydrieraustrags | Umsatz [%] | Selektivität NPG [%] |
|---|---|---|---|
| V1 | 6,4 | 84,8 | 98,4 |
| V2 | 6,9 | 91,9 | 99,2 |
| 1 | 7,8 | 97,1 | 99,9 |
| 2 | 8,3 | 97,2 | 99,7 |
| 3 | 8,6 | 97,6 | 99,6 |
| 4 | 8,9 | 97,9 | 99,3 |

| | | | |
|---|---|---|---|
| NPG = Neopentylglykol | | | |

### Beispiel 2

Hydrierung von Dimethylolbutanal (DMB) zu Trimethylolpropan (TMP)

### Hydrierfeed

Der Hydrierfeed wurde gemäß Beispiel 6 der PCT/WO 98/28253 hergestellt.

### Katalysatoraktivierung

300 ml eines Cu/TiO₂-Katalysator J PCT/WO 99/44974 wurden in einem Rohrreaktor bei 190°C durch Überleiten eines Gemisches aus 5 Vol.% Wasserstoff und 95 Vol.% Stickstoff (Gesamtvolumen 150 Nl/h) drucklos 24 h aktiviert.

### Hydrierung

Als Ausgangslösung diente das vorstehend als Hydrierfeed beschriebene Gemisch. Dem Gemisch wurden zwischen 0 und 3 Gew.% (bezogen auf den Hydrierfeed) einer 10%igen wässrigen Lösung von Zitronensäure zugesetzt, um den in Tabelle 2 angegebenen pH-Wert des Hydrieraustrags einzustellen. Der so erhaltene Hydrierzulauf wurde in Rieselfahrweise bei 80 bar H₂-Druck über den auf 120°C beheizten Reaktor gefahren. Die Belastung betrug 0,4 kg Dimethylolbutanal (DMB)/ (l_{Kat.}*h). Ein Teil des Hydrieraustrags wurde dem Zulauf wieder zugemischt (Kreislauffahrweise). Das Verhältnis von Kreislauf zu Zulauf betrug 10:1. Tabelle 2 zeigt gemittelte Umsätze und Selektivitäten über mehrere Tage bei unterschiedlichen pH-Werten. Der pH-Wert wurde von Proben des Reaktoraustrags bei Raumtemperatur gemessen.

Einen Vergleich des erfindungsgemäßen Verfahren mit den Vergleichsbeispielen V3 und V4, bei denen der pH-Wert des Hydrieraustrags jeweils außerhalb des erfindungsgemäßen Bereichs liegt, zeigt Tabelle 2.

Zur pH-Wert-Messung wurde ein pH-Meter der Firma Knick Modell 766 mit einer Glaselektrode N1041A der Firma Schott verwendet.

**Tabelle 2**

| Beispiel | pH-Wert des Hydrieraustrags | Umsatz [%] | Selektivität TMP [%] |
|---|---|---|---|
| 5 | 7,4 | 98,9 | 95,7 |
| 6 | 7,2 | 97,8 | 94,0 |
| 7 | 7,1 | 97,2 | 93,4 |
| 8 | 7,0 | 95,4 | 92,6 |
| V3 | 5,1 | 83,8 | 92,3 |
| V4 | 4,5 | 81,2 | 92,8 |

| | | | |
|---|---|---|---|
| TMP = Trimethylolpropan | | | |

## Patentansprüche

1. Verfahren zur katalytischen Hydrierung von Methylolalkanalen der allgemeinen Formel 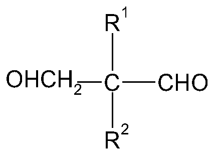 in der R¹ und R² unabhängig voneinander eine weitere Methylolgruppe oder eine Alkylgruppe mit 1 bis 22 C-Atomen oder eine Aryl- oder Aralkylgruppe mit 6 bis 33 C-Atomen bedeuten, in der Flüssigphase an einem Hydrierkatalysator, **dadurch gekennzeichnet, dass** durch Zusatz mindestens eines tertiären Amins, einer anorganischen Base oder einer anorganischen oder organischen Säure zum Hydrierfeed ein pH-Wert von 7,0 bis 9,0 im Hydrieraustrag eingestellt wird.

2. Verfahren nach Anspruch 1 **dadurch gekennzeichnet, dass** der Hydrierfeed weniger als 5 Gew.-% Formaldehyd aufweist

3. Verfahren nach Anspruch 1 oder 2 **dadurch gekennzeichnet, dass** ein Tri-n-alkylamin verwendet wird.

4. Verfahren nach einem der Ansprüche 1 bis 3 **dadurch gekennzeichnet**, das Trimethylamin, Triethylamin, Tri-n-propylamin und oder Tri-n-butylamin zugesetzt wird.

5. Verfahren nach einem der Ansprüche 1 oder 2 **dadurch gekennzeichnet**, das Essigsäure zugesetzt wird.

6. Verfahren nach einem der Ansprüche 1 bis 4, **dadurch gekennzeichnet, dass** der Hydrierkatalysator mindestens ein Metall der Nebengruppen 8 bis 12 des Periodensystems der Elemente aufweist.

7. Verfahren nach einem der Ansprüche 1 bis 5, **dadurch gekennzeichnet, dass** es sich um einen Trägerkatalysator handelt.

8. Verfahren nach Anspruch 6, **dadurch gekennzeichnet, dass** als Trägermaterial die Oxide des Titans, Zirkoniums, Hafniums, Siliciums und/oder Aluminiums verwendet werden.

9. Verfahren nach einem der Ansprüche 5 bis 7, **dadurch gekennzeichnet, dass** der Hydrierkatalysator Kupfer auf einem Aluminiumoxid- oder Titandioxid-haltigen Trägermaterial in An- oder Abwesenheit eines oder mehrerer der Elemente Magnesium, Barium, Zink oder Chrom aufweist.

10. Verfahren nach einem der Ansprüche 1 bis 8, **dadurch gekennzeichnet, dass** es sich um Hydroxypivalinaldehyd, Pentaerythrose oder Dimethylolbutanal handelt.

## Claims

1. A process for catalytically hydrogenating methylolalkanals of the general formula 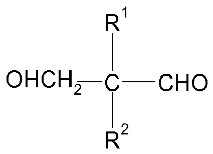 in which R¹ and R² are each independently a further methylol group or an alkyl group having from 1 to 22 carbon atoms or an aryl or aralkyl group having from 6 to 33 carbon atoms, in the liquid phase over a hydrogenation catalyst, which comprises setting a pH of from 7.0 to 9.0 in the hydrogenation effluent by adding at least one tertiary amine, an inorganic base or an inorganic or organic acid to the hydrogenation feed.

2. The process according to claim 1, wherein the hydrogenation feed comprises less than 5% by weight of formaldehyde.

3. The process according to claim 1 or 2, wherein a tri-n-alkylamine is used.

4. The process according to any of claims 1 to 3, wherein trimethylamine, triethylamine, tri-n-propylamine and/or tri-n-butylamine is added.

5. The process according to either of claims 1 and 2, wherein acetic acid is added.

6. The process according to any of claims 1 to 4, wherein the hydrogenation catalyst comprises at least one metal of transition groups 8 to 12 of the Periodic Table of the Elements.

7. The process according to any of claims 1 to 5, which comprises a supported catalyst.

8. The process according to claim 6, wherein the support material used comprises the oxides of titanium, zirconium, hafnium, silicon and/or aluminum.

9. The process according to any of claims 5 to 7, wherein the hydrogenation catalyst comprises copper on an alumina or titania support material in the presence or absence of one or more of the elements magnesium, barium, zinc or chromium.

10. The process according to any of claims 1 to 8, which comprises hydroxypivalaldehyde, pentaerythrose or dimethylolbutanal.

## Revendications

1. Procédé d'hydrogénation catalytique de méthylolalcanals de formule générale : 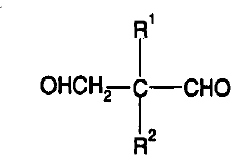 dans laquelle R¹ et R² signifient indépendamment l'un de l'autre un autre groupe méthylol ou un groupe alkyle avec 1 à 22 atomes de carbone ou un groupe aryle ou aralkyle avec 6 à 33 atomes de carbone, en phase liquide sur un catalyseur d'hydrogénation, **caractérisé en ce que** par l'addition d'au moins une amine tertiaire, d'une base inorganique ou d'un acide inorganique ou organique au mélange d'alimentation d'hydrogénation, on règle un pH compris entre 7,0 et 9,0.

2. Procédé selon la revendication 1, **caractérisé en ce que** le mélange d'alimentation d'hydrogénation présente moins de 5 % en poids de formaldéhyde.

3. Procédé selon la revendication 1 ou 2, **caractérisé en ce qu'**on utilise une tri-n-alkylamine.

4. Procédé selon l'une des revendications 1 à 3, **caractérisé en ce qu'**on ajoute la triméthylamine, la triéthylamine, la tri-n-propylamine ou la tri-n-butylamine.

5. Procédé selon l'une des revendications 1 ou 2, **caractérisé en ce qu'**on ajoute l'acide acétique.

6. Procédé selon l'une des revendications 1 à 4, **caractérisé en ce que** le catalyseur d'hydrogénation présente au moins un métal des sous-groupes 8 à 12 du système périodique des éléments.

7. Procédé selon l'une des revendications 1 à 5, **caractérisé en ce qu'**il s'agit d'un catalyseur supporté.

8. Procédé selon la revendication 6, **caractérisé en ce qu'**on utilise comme matériau de support les oxydes du titane, du zirconium, de l'hafnium, du silicium et/ou de l'aluminium.

9. Procédé selon l'une des revendications 5 à 7, **caractérisé en ce que** le catalyseur d'hydrogénation présente du cuivre sur un matériau de support contenant de l'oxyde d'aluminium ou du dioxyde de titane en présence ou en l'absence d'un ou de plusieurs des éléments magnésium, baryum, zinc ou chrome.

10. Procédé selon l'une des revendications 1 à 8, **caractérisé en ce qu'**il s'agit d'hydroxypivalinaldéhyde, de pentaérythrose ou de diméthylolbutanal.
